# EUROPÄISCHE PATENTSCHRIFT

(11) **EP 0 799 212 B1**
(45) Veröffentlichungstag und Bekanntmachung des Hinweises auf die Patenterteilung: **27.01.1999**
(21) Anmeldenummer: 95942661.0
(22) Anmeldetag: 13.12.1995
(51) Int. Cl.: C07D 251/60, C07D 251/62

(54) **VERFAHREN ZUR HERSTELLUNG VON HOCHREINEM MELAMIN**
PROCESS FOR PRODUCING HIGH-PURITY MELAMINE
PROCEDE DE PRODUCTION DE MELAMINE TRES PURE

(30) Priorität: 23.12.1994 AT 2392/94
(43) Veröffentlichungstag der Anmeldung: 08.10.1997
(73) Patentinhaber: Agrolinz Melamin GmbH, A-4021 Linz (AT)
(72) Erfinder: CANZI, Lorenzo, I-20131 Mailand (IT); CANZI, Aldo, I-20129 Mailand (IT); COUFAL, Gerhard, I-22070 Appiano Gentile (IT); GIACOMUZZO, Silvano, I-21012 Cassano Magnago (IT); VIRARDI, Mario, I-20025 Legnano (IT); MÜLLNER, Martin, A-4020 Linz (AT)
(74) Vertreter: Kunz, Ekkehard, Dr.
(86) Internationale Anmeldenummer: EP9504919
(87) Internationale Veröffentlichungsnummer: WO9620182

(56) Entgegenhaltungen:
- EP-A- 0 612 560
- WO-A-95/01345
- GB-A- 800 722
- US-A- 3 116 294
- US-A- 3 484 440
- US-A- 3 637 686
- ULLMANN's ENCYCLOPEDIA OF INDUSTRIAL CHEMISTRY, 5. Ausgabe, Band A16, 1990, VCH Verlagsgesellschaft, Weilheim (DE); Seiten 174-179

## Beschreibung

Aus der Literatur ist bereits eine Vielzahl von Verfahren zur Herstellung von Melamin bekannt. Ein bevorzugtes Ausgangsmaterial ist dabei Harnstoff, der entweder bei hohem Druck und nichtkatalytisch oder bei niedrigem Druck und unter Verwendung eines Katalysators zu Melamin, Ammoniak und CO₂ umgesetzt wird.

Die bekannten Hochdruckverfahren, etwa nach Melamine Chemicals, Montedison oder Nissan, bei denen das Melamin zuerst als Flüssigkeit gebildet wird, haben zwar einen im Vergleich zu Niederdruckverfahren geringeren Energieverbrauch, Melamin enthält jedoch, falls keine Reinigungsstufen vorhanden sind, Verunreinigungen wie Melam, Melem, Ammelin, Ammelid oder Ureidomelamin, die bei manchen Weiterverarbeitungen des Melamins stören.

Die Aufarbeitung des durch ein Hochdruckverfahren hergestellten Melamins erfoglt beispielsweise nach US 4 565 867 (Melamine Chemicals) durch Abtrennung der CO₂- und NH₃-Abgase vom flüssigen Melamin, wobei der Druck und die Temperatur auf den gleichen Werten, wie sie im Reaktor vorliegen, gehalten werden, worauf das flüssige Melamin einer Produktkühleinheit zugeführt wird, entspannt und mit einem flüssigen Medium, etwa flüssigem, wasserfreiem Ammoniak rasch gekühlt bzw. abgeschreckt wird (Quenchen).

Gemäß US 3,116,294 (Montecatini) werden ebenfalls zuerst die CO₂- und NH₃-Abgase abgetrennt, das flüssige Melamin wird, um noch gelöstes CO₂ zu entfernen, im Gegenstrom mit NH₃ behandelt, in einem weiteren Reaktor gesammelt und eine bestimmte Zeit darin verweilen gelassen. Zuletzt wird Melamin aus dem zweiten Reaktor entnommen und durch Abschrecken mit Wasser oder durch Mischen mit kalten Gasen rasch abgekühlt.

Die Reinheit von Melamin, das durch eines dieser Verfahren gewonnenen wird, ist jedoch für viele Anwendungen, etwa bei der Herstellung von Melamin-Formaldehydharzen für Oberflächenbeschichtungen, nicht ausreichend, da insbesondere der Gehalt an Melem zu hoch ist.

Gemäß US 3,637,686 (Nissan) wird die durch thermische Zersetzung von Harnstoff erhaltene rohe Melaminschmelze schnell mit flüssigem NH₃ oder kaltem NH₃-Gas auf 200 bis 270°C abgekühlt, und in einem zweiten Schritt mit wäßriger NH₃-Lösung auf 100 bis 200°C weiter abgekühlt. Anschließend muß, um eine zufriedenstellende Reinheit des Melamins zu erreichen, umkristallisiert werden.

In EP-A-0612560 ist ein Hochdruck-Melamin-Reaktor beschrieben, wobei es Erfindungsziel ist, sämtliche Apparate, die in den bekannten industriellen Hochdruckprozessen notwendig sind, um Melamin herzustellen, in einem einzigen Hochdruckreaktor zu vereinen. Allerdings kann dabei nur rohes Melamin hergestellt werden.
In GB-A-0800722 ist ein Sublimationsverfahren beschrieben, nach welchem dehydratisierter Harnstoff bei 350 bis 450 °C und 20 bis 100 atm Druck in Gegenwart von Ammoniak durch einen Reaktor geleitet und anschließend sublimiert wird.
US-A-3 484 440 beschreibt ebenfalls ein Sublimationsverfahren, bei dem das flüssige Melamin vor der Sublimation über eine Stunde lang bei einem Druck von 40 - 100 kg/cm² und einer Temperatur von 420 - 480 °C zusammen mit den Offgasen gehalten wird. Unter diesen Bedingungen werden einerseits zuerst die Nebenprodukte in der Melaminschmelze laufend in Melamin umgewandelt, andererseits wird aus der darüberliegenden Gasphase, die mit Melamin gesättigt ist, Melamin laufend entfernt und im Separator mit Hilfe von Wasserdampf bei 150 °C in fester Form abgeschieden. Ohne kontinuierlichen Abzug des gasförmigen Melamins ist es jedoch nicht möglich, hochreines Melamin herzustellen, da sich die Nebenprodukte akkumulieren würden.

Aufgabe der vorliegenden Erfindung war es demnach, ein Verfahren zu finden, das die Herstellung von hochreinem Melamin ohne zusätzliche Reinigungsstufen mit einer Reinheit von über 99,8 % und einen Melemgehalt von unter 100 ppm ermöglicht .

Unerwarteterweise konnte diese Aufgabe durch ein Verfahren gelöst werden, bei dem flüssiges Melamin in der letzten Stufe langsam, bzw. kontrolliert abgekühlt wird.

Gegenstand der vorliegenden Erfindung ist demnach ein Verfahren zur Herstellung von hochreinem Melamin ausgehend von einer unter Druck durchgeführten Harnstoffumsetzung, das dadurch gekennzeichnet ist, daß anschließend an die Umsetzungsreaktion eine Nachbehandlung des Melamins bestehend aus den Schritten
a) Abtrennung des NH₃/CO₂-Gasgemisches vom flüssigen Melamin, gegebenenfalls
b) Reduktion des im flüssigen Melamin gelösten CO₂ durch Einbringen von gasförmigem Ammoniak
c) Verweilenlassen des flüssigen Melamins für eine mittlere Verweilzeit von bis zu 8 Stunden bei einer Temperatur zwischen 430°C und dem Schmelzpunkt von Melamin und einem Ammoniakpartialdruck von 50 bis 400 bar und
d) langsames, kontrolliertes Abkühlen durch Erniedrigen der Temperatur von der in a), b) bzw. c) vorhandenen Temperatur auf 330 bis 270°C mit einer Kühlrate bis zu 150°C/min bei einem Ammoniakpartialdruck von 50 bis 400 bar, wobei höhere Drücke schnellere Kühlraten erlauben und umgekehrt niedrigere Drücke langsamere Kühlraten erfordern,
durchgeführt wird, worauf in beliebiger Reihenfolge entspannt, auf Raumtemperatur abgekühlt und hochreines Melamin in Pulverform erhalten wird.

Das erfindungsgemäße Verfahren eignet sich zur Reinigung von Melamin, das aus Harnstoff gemäß einem der bekannten Hochdruckverfahren, wie etwa nach dem Melamine Chemical-, Montedison- oder Nissanprozeß, wie beispielsweise in Ullmann's Encyclopedia of Industrial Chemistry, 5^{th} Edition, Vol. A16, pp 174-179 beschrieben, erhalten wird. Die Harnstoffumsetzung erfolgt nach diesen Verfahren zumeist in einem Temperaturbereich von 370 bis 430°C und einem Druck von etwa 70 bis 250 bar. Das dabei entstehende Melamin wird schließlich als flüssige Phase erhalten.

Nach dem erfindungsgemäßen Verfahren wird im Schritt a) das im Reaktor gebildete Reaktionsgemisch, bestehend aus der flüssigen Melaminphase und einer CO₂/NH₃-Gasphase, in einer geeigneten Apparatur, beispielsweise in einem Gasseparator, aufgetrennt, bzw. es wird die Gasphase von der flüssigen Phase abgetrennt. Der Separator wird dabei auf einer Temperatur oberhalb des Schmelzpunktes von Melamin gehalten, vorzugsweise sind Temperatur und Druck etwa gleich wie im Reaktor. Das CO₂/NH₃-Gasgemisch, das noch Melamin enthält, wird über Kopf abgezogen und in bekannter Weise, etwa durch Einleiten in einen Wäscher, aufgearbeitet und wieder eingesetzt.
Anschließend an die Gasabtrennung bzw. zur gleichen Zeit kann gasförmiges NH₃ eingeleitet werden, wodurch das im Melamin gelöste CO₂ reduziert wird (Schritt b). Die Temperatur liegt dabei wiederum auf einem Wert oberhalb des Schmelzpunktes von Melamin, vorzugsweise sind Temperatur und Druck etwa auf den gleichen Werten wie im Reaktor.
Ob NH₃ eingeleitet wird, sowie die Dauer des Einbringens des gasförmigen NH₃ und die Menge an NH₃ hängen vom gewünschten Endwert des im Melamin gelösten CO₂ ab. Das Ammoniak kann dabei entweder in die gasförmige Phase oder direkt in die flüssige Melaminphase eingebracht werden.

Im nächsten Schritt wird das flüssige Melamin gegebenenfalls für einige Zeit in Anwesenheit von Ammoniak stehen- bzw. verweilengelassen. Hiebei ist es auch möglich andere Gase, z. B. Stickstoff, zuzumischen. Die mittlere Verweilzeit beträgt dabei 0 bis 8 Stunden, unter Umständen sind jedoch auch höhere Verweilzeiten möglich. Bevorzugt beträgt die Verweilzeit 10 Minuten bis 4 Stunden. Während dieser Zeit wird ein Ammoniakpartialdruck von 50 bis 400 bar, bevorzugt von 70 bis 200 bar, eingestellt. Der Druck in Schritt (c) kann dabei auch auf einen höheren Wert als im Reaktor eingestellt werden. Die Temperatur liegt dabei bei einem Wert zwischen dem Schmelzpunkt von Melamin und 430°C, bevorzugt zwischen dem Schmelzpunkt von Melamin und 400°C.

Anschließend an Schritt c) bzw. b) oder a) erfolgt das langsame bzw. kontrollierte Abkühlen des flüssigen Melamins. Dabei wird das flüssige Melamin von der im Schritt c) bzw. b) oder a) vorhandenen Temperatur auf eine Temperatur von zwischen 270° bis 330°C, bevorzugt von einer Temperatur von etwa 370° auf eine Temperatur bis zu etwa 290°C, mit einer definierten Kühlrate abgekühlt. Die Kühlrate kann bis zu 150°C/min, bevorzugt bis zu 100°C/min, besonders bevorzugt bis zu 40°C/min betragen. Die untere Grenze der Kühlrate ist abhängig von technischen und ökonomischen Gegebenheiten. Sie kann dabei, je nach den vorhandenen technischen und ökonomischen Gegebenheiten, beliebig klein gewählt werden. Schritt d) wird, wie die vorangegangenen Schritte, in Anwesenheit von Ammoniak durchgeführt. Der Ammoniakpartialdruck beträgt dabei 50 bis 400 bar, bevorzugt etwa 70 bis 200 bar. Es kann auch wiederum ein höherer Druck als im Reaktor eingestellt werden.
Die einzustellende Kühlrate ist dabei abhängig vom herrschenden Ammoniakpartialdruck, wobei höhere Drücke schnellere Kühlraten erlauben und umgekehrt niedrigere Drücke langsamere Kühlraten erfordern. Die Kühlrate kann gegebenenfalls in dem kontrolliert abzukühlenden Bereich variieren, wobei keine konstante Kühlrate, sondern ein definiertes Kühlprogramm eingestellt werden kann. Unter einem definierten Kühlprogramm sind dabei verschiedene Kühlvarianten zu verstehen, bei welchen die Kühlrate bei verschiedenen Temperaturen unterschiedliche Werte einnehmen kann. Dabei ist es auch möglich, den Druck zu variieren. Es kann beispielsweise zu Beginn von Schritt d) über eine gewisse Zeit eine konstante Temperatur eingestellt und anschließend mit einer in Abhängigkeit vom Druck gewählten Kühlrate auf den gewünschten Temperaturendwert abgekühlt werden. Eine andere mögliche Variante ist beispielsweise ein Abwechseln von Haltephasen, bei welchen die Temperatur eine gewisse Zeit auf der gerade herrschenden Temperatur gehalten wird, und Abkühlphasen. Auch können langsame und schnellere Abkühlphasen in wechselnder Reihenfolge eingestellt werden. Die Abkühlphase kann gewünschtenfalls auch durch einfaches Abschalten der Heizung und Stehenlassen bei Raumtemperatur durchgeführt werden, wodurch ein langsames, exponentielles Abkühlen des flüssigen Melamins bis zu einer vorgegebenen Temperatur erzielt wird. Das Kühlprogramm weist somit mehrere unterschiedliche Varianten auf und kann je nach gewünschtem Endwert an Verunreinigungen und in Abhängigkeit von dem gewählten Verfahrensablauf den jeweiligen Gegebenheiten angepaßt werden.
Oberhalb des langsam bzw. kontrolliert abzukühlenden Bereiches (d) d.h. insbesondere oberhalb des bevorzugten Bereiches, in welchem Schritt d) durchgeführt wird, nämlich oberhalb von etwa 370°C kann je nach den jeweiligen Gegebenheiten, wie etwa Verfahrensablauf oder apparative Ausstattung, entweder langsam, d. h. mit einer Kühlrate bis zu 150°C/min in Abhängigkeit vom herrschenden Druck, oder auch schneller abgekühlt werden.
Unterhalb des kontrolliert abzukühlenden Bereich d. h. unterhalb von 330 bis 270°C, kann die Reaktionsapparatur entspannt und Melamin mit einer beliebigen Kühlrate auf Raumtemperatur abgekühlt werden, worauf hochreines pulverförmiges Melamin erhalten wird. Es kann jedoch auch je nach den technischen Gegebenheiten zuerst abgekühlt und anschließend entspannt werden.
Die Schritte a) bis d) können bei dem erfindungsgemäßen Verfahren gegebenenfalls in getrennten, für den jeweiligen Schritt geeigneten Behältern bzw. Apparaturen durchgeführt werden.
Es sind jedoch auch andere Varianten möglich. So können beispielsweise die Schritte a) und b), sowie die Schritte c) und d) jeweils gemeinsam in der gleichen Apparatur durchgeführt werden.
Eine weitere Möglichkeit besteht darin, daß anschließend an den Schritt a) das Melamin in einen Verweilzeitbehälter übergeführt wird, in welchem die Schritte b) und c) durchgeführt werden und daß Schritt d) in einem getrennten Behälter erfolgt. Die Kombination von Schritt a) bis c) in einer gemeinsamen Apparatur mit anschließender Kühlvorrichtung für Schritt d) ist ebenfalls eine mögliche Verfahrensvariante. Die Prozeßführung muß jedoch den jeweiligen Gegebenheiten, d. h. je nach den Anlagen zur Harnstoffumsetzung, den räumlichen Gegebenheiten, dem geplanten Zeitbedarf für Abkühlphase, der Verweilzeit und anderen Faktoren, angepaßt werden.
Das erfindungsgemäße Verfahren kann je nach Bedarf sowohl diskontinuierlich als auch kontinuierlich durchgeführt werden.

Das erfindungsgemäße Verfahren eignet sich jedoch auch, in etwas abgewandelter Form, zur Reinigung von verunreinigtem Melamin, das aus einem beliebigen aus dem Stand der Technik bekannten Prozeß anfällt und Verunreinigungen, wie Ammelin, Ammelid, Melam, Melem oder Ureidomelamin enthält. Es muß daher nicht zwingend an eine Melaminanlage gekoppelt werden. Es kann daher auch Mutterlaugenmelamin, das beispielsweise bei bisher üblichen Umkristallisationsverfahren von Melamin anfällt, auf diesem Wege gereinigt werden.

Ein weiterer Gegenstand der vorliegenden Erfindung ist demnach ein Verfahren zur Herstellung von hochreinem Melamin, das dadurch gekennzeichnet ist, daß verunreinigtes Melamin bei einem Ammoniakpartialdruck von 50 bis 400 bar auf eine Temperatur, die zwischen dem Schmelzpunkt von Melamin und 430°C liegt, gebracht wird, das flüssige Melamin für 0 bis 8 Stunden in diesem Temperaturbereich verweilen gelassen wird und anschließend ein langsames, kontrolliertes Abkühlen durchgeführt wird, wobei die Temperatur mit einer Kühlrate bis zu 150°C/min bei einem Ammoniakpartialdruck von 50 bis 400 bar, auf 330 bis 270°C erniedrigt wird, wobei höhere Drücke schnellere Kühlraten erlauben und umgekehrt niedrigere Drücke langsamere Kühlraten erfordern, worauf in beliebiger Reihenfolge entspannt, auf Raumtemperatur abgekühlt und hochreines Melamin in Pulverform erhalten wird.
Durch die erfindungsgemäßen Verfahren wird Melamin mit einer Reinheit von bis zu über 99,8 % erhalten, sodaß weitere Reinigungsschritte wie etwa Umkristallisieren nicht mehr nötig sind. Der Gehalt der einzelnen Verunreinigungen, insbesondere von Melem, kann dabei so niedrig gehalten werden, daß diese Verbindungen bei keiner Art von Weiterverarbeitung des Melamins stören.

### Beispiel 1 bis 5:

x g flüssiges Melamin, erhalten durch Harnstoffumsetzung im industriellen Maßstab bei 375°C und 70 bis 75 bar, wurden in einem Behälter eingetragen. Daraus wurde eine Probe gezogen, rasch abgekühlt und der Anfangsgehalt an Verunreinigungen bestimmt. Nach Abtrennung der NH₃/CO₂-Abgase (Schritt a) wurde das flüssige Melamin für etwa 15 Minuten bei 370°C und 85 bar, mit NH₃ behandelt (Schritt b).
Anschließend wurde das flüssige Melamin etwa 60 bis 90 Minuten bei 370°C und einem Ammoniakdruck von 85 bar verweilen gelassen (Schritt c), und das flüssige Melamin durch Abschalten der Heizung exponentiell auf 280°C abkühlen gelassen, sodaß eine Kühlrate <1 °C/min erreicht wurde (Schritt d). Dann wurde entspannt und langsam auf Raumtemperatur abgekühlt.
Die jeweilige Melaminmenge, der Anfangsgehalt an den Verunreinigungen Ammelin (AN), Ammelid (AD), Melem (ME), Melam (MA) und Ureidomelamin (UM), sowie der Gehalt der Verunreinigungen nach den Schritten a) + b) + c) + d) (Ende) sind aus Tabelle 1 ersichtlich.

**Tabelle 1:**

| Beispiel 1 | Melamin | AN | AD | ME | MA | UM |
|---|---|---|---|---|---|---|
| | (g) | (ppm) | (ppm) | (ppm) | (ppm) | (ppm) |
| Anfang | 1096 | 5700 | 1400 | 17400 | 3000 | 500 |
| Ende | | 100 | <50 | <50 | <300 | <50 |
| | | | | | | |

| Beispiel 2: | | | | | | |
|---|---|---|---|---|---|---|
| Anfang | 1710 | 4600 | 3000 | 5600 | 1500 | 500 |
| Ende | | 100 | 180 | <50 | <300 | <50 |
| | | | | | | |

| Beispiel 3: | | | | | | |
|---|---|---|---|---|---|---|
| Anfang | 410 | 4300 | 1600 | 7200 | 2700 | 500 |
| Ende | | 180 | <50 | <50 | <300 | <50 |
| | | | | | | |

| Beispiel 4: | | | | | | |
|---|---|---|---|---|---|---|
| Anfang | 666 | 4300 | 1000 | 4500 | 1600 | 200 |
| Ende | | 100 | <50 | <50 | <300 | <50 |
| | | | | | | |

| Beispiel 5: | | | | | | |
|---|---|---|---|---|---|---|
| Anfang | 731 | 11400 | 2100 | 8600 | 2300 | 500 |
| Ende | | 130 | <50 | <50 | <300 | <50 |

### Beispiel 6:

1700 g flüssiges Melamin, hergestellt analog Beispiel 1 bis 5, wurden von den NH₃/CO₂-Abgasen abgetrennt, etwa 15 min mit NH₃ bei einem Druck von 84 bar behandelt und 2 Stunden bei 375°C und 85 bar stehengelassen.
Anschließend wurde die Heizung entfernt, das Melamin auf 290°C langsam abkühlen gelassen, entspannt und auf Raumtemperatur abkühlen gelassen. Die Endwerte an Verunreinigungen im Melamin betrugen:

| | | | | | |
|---|---|---|---|---|---|
| AN | 100 ppm | ME | < 50 ppm | UM | < 50 ppm |
| AD | <50 ppm | MA | <300 ppm | | |

### Beispiel 7:

1205 g flüssiges Melamin, hergestellt analog Beispiel 1 bis 5 wurden vom NH₃/CO₂-Abgas abgetrennt, etwa 15 Minuten bei 85 bar mit NH₃ behandelt und ohne Verweilzeit, nach Abschalten der Heizung auf 290°C abkühlen gelassen. Anschließend wurde entspannt und auf Raumtemperatur abgekühlt.
Die Endwerte an Verunreinigungen im Melamin betrugen:

| | | | | | |
|---|---|---|---|---|---|
| AN | 270ppm | MA | < 200ppm | UM | < 100ppm |
| AD | < 50ppm | ME | 580 ppm | | |

### Beispiel 8:

300 g Melamin mit 8100 ppm an Melem und etwa 65 g flüssiges NH₃ wurden auf 360°C erhitzt. Der Ammoniakdruck betrug etwa 80 bar. Anschließend wurde das Melamin bei diesen Bedingungen verweilen gelassen und danach langsam von 360°C auf 330°C in 28 Minuten (Kühlrate etwa 0,8°C/min) abgekühlt. Der Endgehalt an Verunreinigungen in Abhängigkeit von der Verweilzeit h betrug nach Entspannen der Reaktionsapparatur und Abkühlen auf Raumtemperatur:

| h (Std) | ME ppm | MA ppm | ANppm | ADppm |
|---|---|---|---|---|
| 1 | 2250 | <300 | 400 | 50 |
| 2 | 430 | <300 | 320 | <50 |
| 4 | 160 | <300 | 310 | <50 |

### Beispiel 9:

300 g Melamin mit 8100 ppm an Melem und die Menge an flüssigem NH₃, die notwendig ist um einen definierten Druck p zu erzielen, wurden auf 364°C aufgeheizt, 2 Stunden bei diesen Bedingungen verweilen gelassen und von 360 auf 330°C in etwa 5 Minuten (Kühlrate 6°C/min) abgekühlt.
Der Gehalt an Melem in Abhängigkeit vom jeweils eingestellten Ammoniakdruck betrug nach Entspannen der Reaktionsapparatur und Abkühlen auf Raumtemperatur:

| p (bar) | ME ppm |
|---|---|
| 80 | 1520 |
| 115 | 250 |
| 130 | 160 |
| 151 | 110 |

### Beispiele 10:

300 g Melamin mit 10 000 ppm an Melem und 117 g an flüssigem Ammoniak wurden auf 370°C erwärmt.
Der Ammoniakdruck betrug dabei etwa 154 bar. Anschließend wurde das Melamin 2 Stunden unter diesen Bedingungen stehengelassen und von 360 auf 330°C mit einer Kühlrate m abgekühlt.
Der Melemgehalt nach Entspannen der Reaktionsapparatur und Abkühlen auf Raumtemperatur betrug in Abhängigkeit von der Abkühlzeit:

| m (°C/min) | ME ppm |
|---|---|
| 7,5 | 210 |
| 0,8 | 100 |

### Beispiele 11:

90 mg Melamin und die zur Einstellung eines Ammoniakdruckes von 150 bar nötige Ammoniakmenge wurden in einem Autoklaven auf 370°C aufgeheizt, 4 Stunden bei diesen Bedingungen verweilen gelassen und anschließend mit einer durchschnittlichen Kühlrate von 18 und von 36°C/min, die durch einen definierten Luftströme erzielt wurden, auf 290°C abgekühlt. Danach wurde rasch, durch Eintauchen in kaltes Wasser, auf Raumtemperatur abgekühlt und entspannt. Der Anfangs- und Endgehalt an Melem betrug

| | ME(ppm) |
|---|---|
| Anfang | 13000 |
| Ende (18°C/min) | 280 |
| Ende (36°C/min) | 600 |

### Beispiel 12:

124 mg Melamin und die zur Einstellung eines Ammoniakdruckes von 200 bar nötige Ammoniakmenge wurden in einem Autoklaven auf 370°C aufgeheizt, 3 Stunden bei diesen Bedingungen verweilen gelassen und anschließend mit einer durchschnittlichen Kühlrate von etwa 100°C/min auf 320°C abgekühlt. Danach wurde rasch, durch Eintauchen in kaltes Wasser, auf Raumtemperatur abgekühlt und entspannt.
Der Anfangs- und Endgehalt an Melem betrug

| | ME(ppm) |
|---|---|
| Anfang | 13000 |
| Ende | 250 |

### Beispiel 13:

300 g Melamin mit 10 000 ppm Melem und die zur Einstellung eines Ammoniakdruckes von 200 bar nötige Ammoniakmenge wurden in einem Autoklaven auf 370°C aufgeheizt, 2 Stunden bei diesen Bedingungen verweilen gelassen und anschließend mit einer Kühlrate m auf 320°C abgekühlt und dann entspannt. Der Melemgehalt betrug in Abhängigkeit von der Kühlrate:

| m(°C/min) | ME ppm |
|---|---|
| 0,9 | <50 |
| 8 | 120 |

## Patentansprüche

1. Verfahren zur Herstellung von hochreinem Melamin ausgehend von einer unter Druck durchgeführten Harnstoffumsetzung, dadurch gekennzeichnet, daß anschließend an die Umsetzungsreaktion eine Nachbehandlung des Melamins bestehend aus den Schritten
a) Abtrennung des NH₃/CO₂-Gasgemisches vom flüssigen Melamin, gegebenenfalls
b) Reduktion des im flüssigen Melamin gelösten CO₂ durch Einbringen von gasförmigem Ammoniak
c) Verweilenlassen des flüssigen Melamins für eine mittlere Verweilzeit von bis zu 8 Stunden bei einer Temperatur zwischen 430°C und dem Schmelzpunkt von Melamin und einem Ammoniakpartialdruck von 50 bis 400 bar und
d) langsames, kontrolliertes Abkühlen durch Erniedrigen der Temperatur von der in a), b) bzw. c) vorhandenen Temperatur auf 330 bis 270°C mit einer Kühlrate bis zu 150°C/min bei einem Ammoniakpartialdruck von 50 bis 400 bar, wobei höhere Drücke schnellere Kühlraten erlauben und umgekehrt niedrigere Drücke langsamere Kühlraten erfordern,
durchgeführt wird, worauf in beliebiger Reihenfolge entspannt, auf Raumtemperatur abgekühlt und hochreines Melamin in Pulverform erhalten wird.

2. Verfahren nach Anspruch 1, dadurch gekennzeichnet, daß die Verweilzeit in Schritt c) 10 Minuten bis 4 Stunden beträgt.

3. Verfahren nach Anspruch 1, dadurch gekennzeichnet, daß die Temperatur in Schritt-c) zwischen 400°C und dem Schmelzpunkt von Melamin liegt.

4. Verfahren nach Anspruch 1, dadurch gekennzeichnet, daß der Ammoniakpartialdruck in Schritt c) zwischen 70 und 200 bar beträgt.

5. Verfahren nach Anspruch 1, dadurch gekennzeichnet, daß in Schritt d) langsam, mit einer kontrollierten Kühlrate von 370 auf 290°C abgekühlt wird.

6. Verfahren nach Anspruch 1, dadurch gekennzeichnet, daß die Kühlrate in Schritt d) bis zu 40°C/min beträgt.

7. Verfahren nach Anspruch 1, dadurch gekennzeichnet, daß Schritt a) und Schritt b) gleichzeitig durchgeführt werden.

8. Verfahren nach Anspruch 1, dadurch gekennzeichnet, daß Schritt a) und Schritt b) nacheinander durchgeführt werden.

9. Verfahren zur Herstellung von hochreinem Melamin, dadurch gekennzeichnet, daß verunreinigtes Melamin bei einem Ammoniakpartialdruck von 50 bis 400 bar auf eine Temperatur, die zwischen dem Schmelzpunkt von Melamin und 430°C liegt, gebracht wird, das flüssige Melamin bis zu 8 Stunden in diesem Temperaturbereich verweilen gelassen wird und anschließend ein langsames, kontrolliertes Abkühlen durchgeführt wird, wobei die Temperatur mit einer Kühlrate bis zu 150°C/min bei einem Ammoniakpartialdruck von 50 bis 400 bar auf 330 bis 270°C erniedrigt wird, wobei höhere Drücke schnellere Kühlraten erlauben und umgekehrt niedrigere Drücke langsamere Kühlraten erfordern, worauf in beliebiger Reihenfolge entspannt, auf Raumtemperatur abgekühlt und hochreines Melamin in Pulverform erhalten wird.

## Claims

1. Process for the production of highly pure melamine starting from a urea conversion carried out under pressure, characterized in that, following the conversion reaction, an after-treatment of the melamine is carried out, comprising the steps
a) separating off the NH₃/CO₂ gas mixture from the liquid melamine, if appropriate
b) reducing the CO₂ dissolved in the liquid melamine by introducing gaseous ammonia
c) allowing the liquid melamine to remain for a mean residence time of up to 8 hours at a temperature between 430°C and the melting point of melamine and an ammonia partial pressure of 50 to 400 bar and
d) slow, controlled cooling by lowering the temperature from the temperature present in a), b) and/or c) to 330 to 270°C at a cooling rate of up to 150°C/min at an ammonia partial pressure of 50 to 400 bar, higher pressures permitting more rapid cooling rates and, vice versa, lower pressures requiring slower cooling rates,
whereupon in any desired sequence the reaction vessel is depressurized and cooled to room temperature and highly pure melamine is obtained in powder form.

2. Process according to Claim 1, characterized in that the residence time in step c) is 10 minutes to 4 hours.

3. Process according to Claim 1, characterized in that the temperature in step c) is between 400°C and the melting point of melamine.

4. Process according to Claim 1, characterized in that the ammonia partial pressure in step c) is between 70 and 200 bar.

5. Process according to Claim 1, characterized in that the reaction vessel is cooled in step d) from 370 to 290°C slowly at a controlled cooling rate.

6. Process according to Claim 1, characterized in that the cooling rate in step d) is up to 40°C/min.

7. Process according to Claim 1, characterized in that step a) and step b) are carried out simultaneously.

8. Process according to Claim 1, characterized in that step a) and step b) are carried out sequentially.

9. Process for the production of highly pure melamine, characterized in that contaminated melamine at an ammonia partial pressure of 50 to 400 bar is brought to a temperature which is between the melting point of melamine and 430°C, the liquid melamine is allowed to remain in this temperature range for up to 8 hours and then a slow, controlled cooling is carried out, the temperature being decreased to 330 to 270°C at a cooling rate of up to 150°C/min at an ammonia partial pressure of 50 to 400 bar, higher pressures permitting more rapid cooling rates and, conversely, lower pressures requiring slower cooling rates, whereupon in any desired sequence the reaction vessel is depressurized and cooled to room temperature and highly pure melamine is obtained in powder form.

## Revendications

1. Procédé de préparation de mélamine très pure à partir d'une transformation d'urée effectuée sous pression, caractérisé en ce que, directement après la réaction de transformation, on effectue un post-traitement de la mélamine, comprenant les étapes suivantes :
a) séparation du mélange gazeux de NH₃/CO₂ de la mélamine liquide,
b) éventuellement, réduction du CO₂ dissous dans la mélamine liquide par introduction de gaz ammoniac,
c) repos de la mélamine liquide pendant un temps de séjour moyen allant jusqu'à 8 heures, à une température comprise entre 430°C et le point de fusion de la mélamine et à une pression partielle d'ammoniac de 50 à 400 bars, et
d) refroidissement lent et maîtrisé par abaissement de la température, passant de la température présente en a), b) ou c) à une température de 330 à 270°C, à une vitesse de refroidissement allant jusqu'à 150°C/min, à une pression partielle d'ammoniac de 50 à 400 bars, des pressions assez élevées permettant des vitesses de refroidissement plus grandes, et inversement, des pressions assez basses nécessitant des vitesses de refroidissement plus petites,
après quoi, dans un ordre quelconque, on relâche la pression, on effectue un refroidissement à la température ambiante et on obtient une mélamine très pure à l'état pulvérulent.

2. Procédé selon la revendication 1, caractérisé en ce que le temps de séjour pendant l'étape c) est de 10 minutes à 4 heures.

3. Procédé selon la revendication 1, caractérisé en ce que la température pendant l'étape c) est comprise entre 400°C et le point de fusion de la mélamine.

4. Procédé selon la revendication 1, caractérisé en ce que la pression partielle d'ammoniac pendant l'étape c) est comprise entre 70 et 200 bars.

5. Procédé selon la revendication 1, caractérisé en ce que, dans l'étape d), on effectue un refroidissement lent à une vitesse de refroidissement maîtrisée de 370 à 290°C.

6. Procédé selon la revendication 1, caractérisé en ce que, dans l'étape d), la vitesse de refroidissement vaut jusqu'à 40°C/min.

7. Procédé selon la revendication 1, caractérisé en ce qu'on effectue simultanément l'étape a) et l'étape b).

8. Procédé selon la revendication 1, caractérisé en ce qu'on effectue successivement l'étape a) et l'étape b).

9. Procédé de préparation de mélamine très pure, caractérisé en ce que, à une pression partielle d'ammoniac de 50 à 400 bars, on amène de la mélamine impure à une température comprise entre le point de fusion de la mélamine et 430°C, on laisse reposer la mélamine liquide dans cette plage de températures pendant un laps de temps allant jusqu'à 8 heures, et ensuite, on effectue un refroidissement lent et maîtrisé, la température étant ramenée à une valeur de 330 à 270°C, à une vitesse de refroidissement allant jusqu'à 150°C/min et à une pression partielle d'ammoniac de 50 à 400 bars, des pressions assez élevées permettant des vitesses de refroidissement plus grandes, et inversement, des pressions assez basses nécessitant des vitesses de refroidissement plus petites, après quoi, dans un ordre quelconque, on relâche la pression, on effectue un refroidissement à la température ambiante et on obtient une mélamine très pure à l'état pulvérulent.
